# EUROPEAN PATENT SPECIFICATION

(11) **EP 1 278 064 B1**
(45) Date of publication and mention of the grant of the patent: **02.07.2008**
(21) Application number: 01810715.1
(22) Date of filing: 18.07.2001
(51) Int. Cl.: G01N 33/543, G01N 27/403, G01N 33/487

(54) **High-density microelectrode arrangement**
Mikroelektrodenanordnung mit hoher Dichte
Agencement de micro-électrodes à haute densité

(43) Date of publication of application: 22.01.2003
(73) Proprietor: CSEM Centre Suisse d'Electronique et de Microtechnique SA, 2007 Neuchâtel (CH)
(72) Inventor: Berdondini, Lucas, 1003 Lausanne (CH); Overstolz, Thomas, 2000 Neuchâtel (CH); Koudelka-Hep, Milena, 2000 Neuchâtel (CH); Seitz, Peter, 8902 Urdorf (CH)
(74) Representative: Schneider Feldmann AG Patent- und Markenanwälte

(56) References cited:
- WO-A-01/33207
- US-A- 5 234 566
- US-A- 5 965 452
- KAKEROW R ET AL: "A MONOLITHIC SENSOR ARRAY OF INDIVIDUALLY ADDRESSABLE MICROELECTRODES" SENSORS AND ACTUATORS A, ELSEVIER SEQUOIA S.A., LAUSANNE, CH, vol. A43, no. 1/3, 1 May 1994 (1994-05-01), pages 296-301, XP000454125 ISSN: 0924-4247

## Description

### Field of the invention

This invention relates to a microelectrode arrangement according to the preamble of the first claim. It is applicable for measuring two-dimensional distributions of voltages or currents with high spatial resolution (a few tens of micrometers) and high temporal resolution (several 100 kHz or MHz). Preferred fields of applications are in the bio-medical area, in pharmacology (e.g., drug screening) and in electrophysiology research on electrogenic biological networks, where high spatio-temporal resolution and low-noise potentiometric or amperometric long-term extracellular recordings are essential.

### Background of the invention

At present, the enabling technologies for the fabrication of microelectrode arrays (MEAs) can be considered as well established. MEA devices allow to electrically contact a network of biological cells at many locations and to study their electrophysiological activity in vivo or in vitro. Several MEAs, especially for in-vitro applications, of various materials and geometries are available and some of them also commercialised (e.g., "MEA60" by Multichannel Systems, "MED Probe" by Panasonic®). They typically comprise an array of 30 to 128 planar or three-dimensional metal (Au, Pt, ITO, TiN) electrodes on a silicon or glass substrate. The chip surface is insulated by biocompatible materials (PA, SiₓN_{y}, EPON SU-8). The electrode diameter generally ranges between 10 µm and 100 µm, and the electrode separation between 100 µm and 200 µm. Patterned metal leads individually electrically connect each electrode to a contact pad, which is electrically connected to an external measuring system. Examples of such systems used with MEAs are described, e.g., in G. W. Gross et al., "A new fixed-array multimicroelectrode system designed for longterm monitoring of extracellular single unit neuronal activity in vitro", Neuroscience Letters, vol. 6, pp. 101-105, 1977, or in S. Martinoia, M. Bove, G. Ciccarelli, M. Grattarola, C. Storment, and G. Kovacs, "A general-purpose system for long-term recordings from a microelectrode array coupled to excitable cells", Journal of Neuroscience Methods, vol. 48, pp. 115-121, 1993.

However, wiring individually each electrode to a pad by patterning metal leads limits the electrode density, the active area, and thus the spatial resolution. In fact, leads connecting the electrodes located in the array center have to pass between the peripheral electrodes, keeping a distance between each lead to avoid signal interference, before contacting the pad. This results in a limited electrode separation in function of the total number of electrodes. For an electrode array of x columns and y lines, x·y leads are necessary as well as x·y pads, resulting in a not adapted technology for thousands of densely packed electrodes. With the present-day technology, it is possible to reduce lead dimensions by improving their patterning resolution, but this has the direct consequence of an increased noise contribution due to their increased resistance. For example, an aluminum lead with a width of 0.5 µm and a thickness of 150 nm has a calculated resistance of 3.56 kΩ/cm. At a bandwidth of 10 MHz and at room temperature, this resistance contributes thermal noise of 24.2 µV/√cm r.m.s. Additionally, lead interconnections pick up noise from the surrounding electromagnetic fields, further increasing the total noise. This is not acceptable when signals of a few tens of µV must be measured. Each electrode, via its contact pad, is individually connected to an external measuring circuit. This results in a complex and large external measuring circuit when thousands of electrodes are integrated.

Another approach replaces the metal electrodes with field-effect transistors (FETs) in order to take advantage of the neuron-silicon adhesion, such as described, e.g., in C. Sprössler, M. Denyer, S. Britland, W. Knoll, and A. Offenhäusser, "Electrical recordings from rat cardiac muscle cells using field-effect transistors", Physical review E., vol. 60, pp. 2171-2176, 1999. The silicon-oxide transistor gate is coupled with the cells resulting in a better cell adhesion than on metal electrodes due to oxide hydrophilic properties. Changes in the cell's membrane voltage cause a variation of the gate voltage, which can be recorded as a difference in source-drain current due to the channel resistance variation.

However, transistor thermal noise contribution (typically 100 µV r.m.s. at a bandwidth of 10 kHz), the individual wiring, exposed to extrinsic noise, between each transistor and the external measuring circuit superimpose an important noise contribution to the recorded signals, limiting the detectable signals to signals greater than 200 µV.

The FET is in this case used as a passive electrode, in the form of a voltage-dependent resistor, without any electrical improvement to the signal-to-noise ratio. The device needs an amplification and filtering circuit for each transistor, in order to achieve a high temporal resolution, and synchronous signal recordings. Moreover, source and drain have to be both accessible for the external measuring circuit, requiring two leads per electrode, and thus limiting the electrode density as previously described for standard metal-electrode arrays. These reasons limit the full integration of the FET arrays and complicate the overall set-up when thousands of active sites have to be integrated in order to achieve a high resolution monitoring of the biological network activity.

Böhm et al. (S. Böhm et al., "The field-effect-addressable Potentiometric Sensor/Stimulator (FAPS): Fabrication and Characterization", Proceedings, Eurosensors XIII, Sept. 12-15, 1999) presented a field-effect-addressable potentiometric sensor/stimulator (FAPS). This device promises a high number of individually addressable, densely packed active sites. Only a non-integrated prototype has been reported consisting in a grid-structure of 5 perpendicular FET-channels (2 mm in length, 100 µm wide) and gate-electrodes on top and below the FET channel. Applying a constant current between the source and the drain and a back-gate voltage creating the channel, it is possible to detect the resistance variation of the channel generated by a surface potential due to the cellular activity.

However, the FET transistor is used as a switch-controlled electrode, and no local amplification is performed. Even though by adding other back-gate electrodes as well as corresponding front-gate electrodes, more addressable points could be realized, the introduction of an external resistance-measuring circuit for each channel results in a complicated electronics set-up for a high number of channels. Moreover, this solution still suffers from noise contributions due to the FET transistors (thermal noise) and from noise pickup on the connecting leads. This introduces a detection limit of signal amplitudes larger than few hundreds of µV, which may finally be decreased to about 100 µV by further improvements.

In order to reduce noise contributions due to the distance between the electrodes and the external amplification stage, and in order to simplify the overall electronic set-up, MEAs, in particular for in-vivo applications, with an on-chip CMOS amplifier circuit placed on the sides of the chip have been developed.

However, this solution only partially solves the interconnection problem, since, as for standard MEAs, electrodes and amplifier inputs are still interconnected by leads, and signals are still contaminated by noise pickup from the surrounding. Additionally, this configuration still limits the electrode density due to the problem of wiring a high number of electrodes: lead dimensions are not allowed to have a cross section smaller than few square microns due to their thermal noise contribution up to several hundreds of µV. Moreover, the complexity of the CMOS amplifier, the required silicon surface and the resulting price significantly increase for a higher number of electrodes, since each electrode needs an input into the measuring circuit.

In the case of a separated signal-treatment chip, the packaging as well as the electrical connection of the chip (containing the electrode array and the integrated measuring circuit) turn out to be critical due to contact noise contributions up to tens of µV. They are also difficult to assemble when a high number of electrodes must be connected. For those reasons, this is not a solution adapted to high-density electrodes arrays.

Burcher et al. (V. Burcher et al., "Electrical properties of a light-addressable microelectrode chip with high electrode density for extracellular stimulation and recording of excitable cells", Biosensors & Bioelectronics, vol. 16, pp. 205-210, 2001) describe a light-addressable microelectrode chip (MEC3600) with 3600 (60×60) electrodes on a surface of 1.8×1.8 mm². The chip is realized with indium-tin oxide lead patterning on a transparent glass substrate, depositing amorphous silicon on the top surface, insulating it with silicon nitride, and, finally, patterning Au or TiN metal electrodes on the nitride. Using the increase in photoconductivity of the amorphous silicon layer upon backside illumination by a focused laser diode, it is possible to opto-electrically connect the electrodes with the corresponding metal lead. Each metal lead, 20 µm wide, is externally connected to an amplification circuit and light addressing is used to select the desired electrode. An optically switched microelectrode array (OSMA) providing 10⁴ spatial patterned electrodes of 50 µm in diameter, fabricated in similar fashion as Burcher, has also been reported.

Even though electrode density is drastically increased with this approach, the spatial electrode resolution is still limited. Firstly, the lateral resolution in the electrolyte is limited by light scattering in the transparent glass substrate. This is responsible for the signal contribution of neighboring electrodes, on the same metal lead, added to the contribution of the selected one. Secondly, a time delay up to some µs between each electrode's recordings is the result of the optical addressing by mechanical displacement time of the light source and of the activation time of the opto-electrical connection between electrodes and leads. No signal amplification is locally performed and noise pickup from the surrounding, before the external amplification, affects the signal-to-noise ratio. Moreover, the opto-electrical connection introduces an additional noise contribution due to charge-pair generation. Both noise contributions result in a detection limit of signals above several hundreds of µV.

An additional important problem in some biological applications is caused by the phototoxicity effect of the addressing light on some cells, especially neurons, rendering this method unsuitable for long-term recordings. The device needs a light source and a light addressing system, as well as a focusing or projecting light system, impeding the full integration of the overall system. Applications needing a full-integrated device, e.g., in-vivo applications, cannot use this device.

WO 01 33207 discloses a recording device having at least one potentiometric electrode, at least one amperometric electrode, wherein both electrodes are located on the same recording device. Also provided is a single locus recording device having at least one potentiometric electrode, at least one amperometric electrode, wherein both electrodes are located on the same recording device.

Patent US5965452 discloses a biologic electrode array and methods for manufacturing and using the same. In one aspect, a matrix of electrodes each coupled to a respective sample-and-hold circuit is provided. The electrodes and sample-and-hold circuits are integral and form an array within a single semiconductor chip, such that each sample-and-hold circuit may be loaded with a predefined voltage provided by a single, timeshared digital-to-analog converter (DAC). Further, all of the sample-and-hold circuits may be accessed through a multiplexer which may be scanned through some or all of the electrode locations. Each sample-and-hold circuit may comprise a capacitor and one or more transistor switches, the switch(es), when closed, providing electrical communication between the capacitor and a source line formed in the matrix.

Patent US 5234566 discloses a biosensor comprising at least one lipid membrane, each membrane including at least one gated ion channel. The membranes comprise a closely packed array of self-assembly amphophilic molecules and the gated ion channel has a conductance which is dependent upon an electric field applied across the membrane. The biosensor of the present invention may comprise a plurality of discrete membranes each including at least one gated ion channel. The conductance of each of the membranes is measurable independently of the conductance of the other membranes.

### Summary of the invention

It is an object of the invention to provide a microelectrode arrangement that overcomes several of the major limitations of the prior art. In particular, the microelectrode arrangement shall combine high spatial resolution, high temporal resolution and good noise performance. This object is solved by the microelectrode arrangement as defined in claim 1.

The invention is inspired by the active-pixel sensor (APS) as used in solid-state image sensors, e.g., for digital photographic cameras. An APS sensor comprises a two-dimensional array of picture elements (pixels). In each pixel the photocharge or photocurrent generated by a photodetector as a function of incident light is actively converted into a voltage or current signal. Each pixel can be addressed and read out individually. APS sensors are realizable with standard complementary metal oxide semiconductor (CMOS) processes.

The idea of the invention is, simply spoken, to replace the photodetectors in an APS by electrodes. The result is an "active-microelectrode" array in which each pixel comprises a contacting electrode and an associated electronic circuit with an amplifier. (The expression "pixel" is used throughout this document for a basic element of the microelectrode arrangement according to the invention, although the microelectrode arrangement does not detect any optical "pictures".) The active-microelectrode array collects charges picked up by the contacting electrodes from electrogenic cells rather than photocharges generated in a photodetector as a function of incident light.

This idea may seem trivial in a retrospective analysis, for a person having knowledge of the invention. This is, however, not a correct approach, as explained in the following. First of all, bio-medical probes and imaging cameras lie in completely different technical fields and do not seem to have anything in common with each other. Consequently it is not at all obvious to seek in the field of imaging cameras for a solution to a problem in bio-medical sensors. Moreover, once the idea is created, its technical realization is difficult and might even seem impossible to a person skilled in the art of APS technology, since especially the production of noble-metal electrodes cannot be simply implemented into the APS manufacturing process.

The microelectrode arrangement according to the invention comprises a plurality of contacting electrodes being adapted for electrically contacting an external surface, and means for amplifying an electric input signal applied to at least one of said contacting electrodes. The microelectrode arrangement further comprises a plurality of microelectrode pixels, each microelectrode pixel comprising a contacting electrode and an amplifier for amplifying an electric input signal applied to said contacting electrode. The amplifier preferably is a differential amplifier, e.g., a programmable differential amplifier. The microelectrode arrangement preferably comprises means for individually addressing each microelectrode pixel.

Compared to arrangements described in the prior art, this configuration simplifies the overall system set-up, resulting in a fully integrated device. Furthermore, the very small electrode-amplifier distance results in a lower influence of environmental noise and in a higher signal to noise ratio. Another advantage lies in the fact that the configuration according to the invention requires x+y leads only, whereas the arrangements according to the prior art require x·y leads.

The microelectrode arrangement according to the invention performs the following tasks:
(a) Detection of a voltage or current signal;
(b) Amplification of the measured signal;
(c) Low-pass filtering for noise reduction; and
(d) Individual addressing of an active electrode for highspeed readout of an arbitrary signal through one or several output amplifiers.

Compared to the prior art, the microelectrode arrangement according to the invention introduces many practically useful properties, some of which are listed in the following:
- It combines high spatial resolution and high temporal resolution, enabling for example the monitoring of the living cell's electrical activity with high spatial and temporal resolution.
- It allows the fabrication of high-density electrode arrays on a large active area, only limited by the size of the wafers used in the semiconductor manufacturing.
- An in-pixel CMOS measuring circuit is integrated for local amplification and filtering, reducing the distance between the electrode and the measuring circuit, which distance is mainly responsible for the noise disturbance, as well as simplifying the overall set-up for high-density electrode arrays. No additional off-chip circuits are necessary for the MEA.
- The on-pixel CMOS measuring circuit can be programmed in three modes resulting in a flexible multi-use device for:
   (i) Potentiometric measurements (in open or closed loop);
   (ii) Amperometric measurements (in open or closed loop); and/or
   (iii) Charge integration measurements for ultra-low-noise current detection (integration mode).
- The measuring circuit offers noise/speed trade-off with a programmable low pass filter and a programmable gain.
- The on-pixel amplifier has a minimum effective input capacitance in order to reduce noise contributions.
- Several tens of MHz of the total-read out speed are possible with present-day silicon (CMOS) technology.
- The arrangement is fabricated with a standard CMOS process followed by a post-processing step.
- An electrode pitch of 10-50 µm is possible with the present-day silicon (CMOS) technology.
- This modular approach offers the possibility to integrate pixels with other functionalities. Temperature sensors, pH sensors, integrated references and/or electrical stimulating pixels can be added to the active recording electrode array. In biological applications this can be used to control or influence the cell growth or survival and the cell activity.
- It offers the possibility to combine outputs from several densely packed electrodes for signal treatment, on-chip or by external hardware or software, in order to improve the signal detection by suitable averaging algorithms.

Finally, the microelectrode arrangement according to the invention offers the possibility to combine outputs from several electrodes for signal treatment, on-chip or by external hardware or software. The high spatial and temporal correlation of the recordings, as well as recordings on several electrodes of the same cellular activity, allow to improve the signal detection (e.g., averaging, signal filtering), or to improve cell activity analysis (e.g., cross-correlation, population vector analysis, linear discriminant analysis, multifactorial statistical analysis, nonlinear discriminant analysis).

Summing up, the arrangement of active microelectrode pixels according to the invention records spatially and temporally resolved images of electric current or voltage distributions. Compared to the state of the art, the active electrode pixels substantially increase the spatial and temporal resolution, while improving the noise performance at the same time. Each active electrode pixel comprises an amplifying circuit that can be operated in three different modes: amperometric measurements of currents, potentiometric measurements of voltages, and charge integration measurements for ultra low-noise current detection. A trade-off between readout noise and speed is obtained by suitably operating low-pass filters in the active electrode pixels. A preferred embodiment employs standard CMOS technology for fabrication, followed by a deposition step of noble metals on the contacting electrodes. Applications include the high-resolution imaging of electrophysiological activity of in-vitro cultured electrogenic cells.

### Brief description of the drawings

The invention is described in greater detail hereinafter relative to the attached schematic drawings.
- Figure 1: shows a perspective view of parts of the microelectrode arrangement according to the invention.
- Figure 2: shows a perspective, partially laid-open view of an application of the microelectrode arrangement according to the invention on in-vitro cultured electrogenic cells.
- Figure 3: shows a circuit diagram of a pixel of the microelectrode arrangement according to the invention.
- Figure 4: shows a timing diagram for an integration mode in which the microelectrode arrangement according to the invention can be operated.

### Description of a preferred embodiment

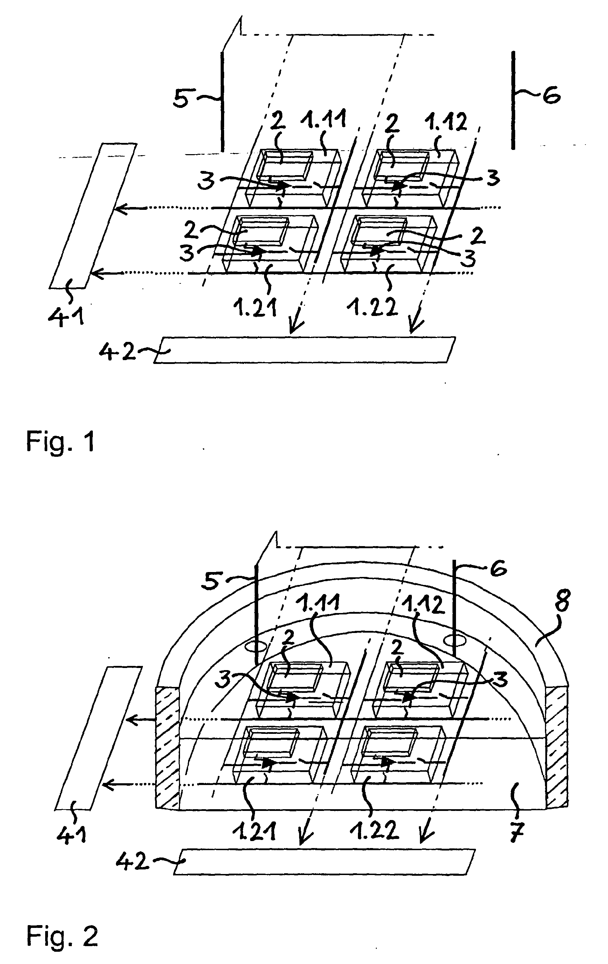

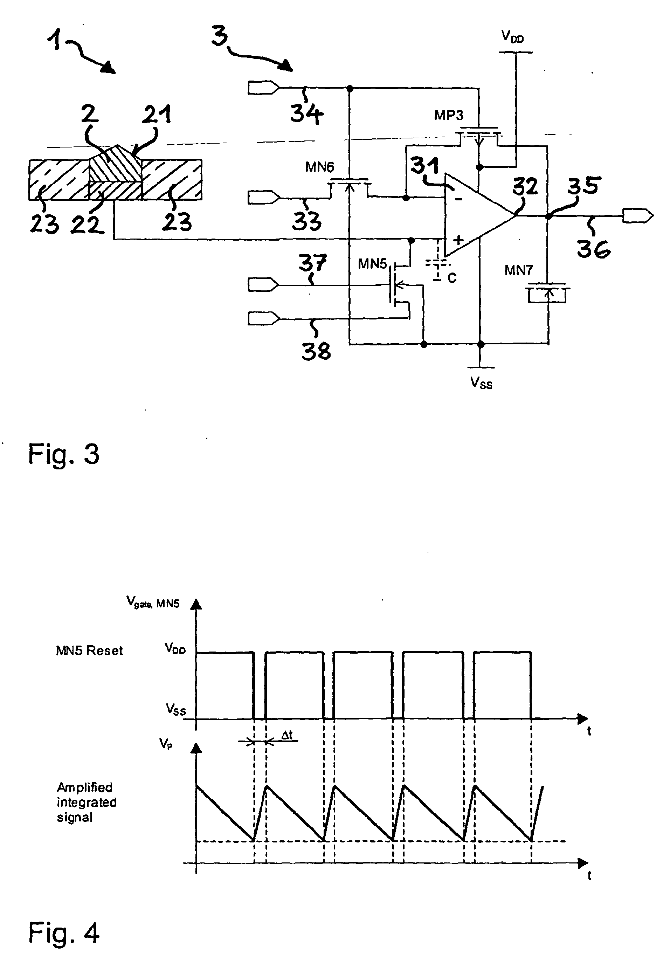

Parts of a preferred embodiment of the microelectrode arrangement according to the invention are shown in Figure 1. The microelectrode arrangement comprises a plurality of microelectrode pixels 1.11, 1.12, ..., 1.21, 1.22, .... Each microelectrode pixel 1.11, 1.12, ..., 1.21, 1.22, ... comprises a contacting electrode 2 and an on-pixel, programmable, differential amplifier 31 (for further details see Fig. 3). The contacting electrode 2 has an active area 21 adapted for electrically contacting an external surface to be monitored, e.g., biological cells. The active area 21 may be flat, as schematically drawn in Figs. 1 and 2, but it alternatively may have any appropriate three-dimensional shape, e.g., be acutely conical as sketched in Fig. 3.

The active area 21 of the contacting electrode 2 is closely placed to the external surface whose voltage or current distribution shall be monitored. The electrical activity is recorded from the integrated electrodes 2 with respect to a reference electrode 5. The locally amplified signals can be read out individually, allowing the reconstruction of an activity image. For this purpose, the arrangement further comprises means 41, 42 for individually addressing and reading out the pixels 1.11, 1.12, ..., 1.21, 1.22, ..., comprising, e.g., a row decoder, a column decoder, a register, an address bus and/or address logic elements. A DC polarization electrode 6 allows to control the DC amplifier polarization, resulting in a programmable gain and bandwidth.

The read-out frame rate of the microelectrode arrangement is proportionally reduced with the number of read pixels 1.11, 1.12, ..., 1.21, 1.22, .... Read-out speed can be improved by integrating multiple signal outputs. Practically, the arbitrary-addressing logic allows firstly to monitor the entire culture activity, and secondly to select certain interesting regions ("regions of interest", ROI). The resulting reduction of the number of read pixels has the consequence of an improved time resolution.

An application of the microelectrode arrangement for in-vitro cultured electrogenic cells is illustrated in **Figure 2**. Electrogenic cells are cultured in a specific culture medium 7 in an appropriate chamber 8, e.g., a Petri dish. The microelectrode arrangement is placed in the immediate vicinity of the culture medium 7, such that the contacting electrodes 1.11, 1.12, ..., 1.21, 1.22, ... are in contact with electrogenic cells, e.g., beneath the culture medium 7. The reference electrode 5, as well as the DC polarization electrode 6, are placed in the same culture medium 7. The electrical activity of the cells is recorded by the contacting electrodes 1.11, 1.12, ..., 1.21, 1.22, ... with respect of the reference electrode 5. By sequentially addressing the pixels, the locally amplified signals are outputted, allowing the reconstruction of an activity image.

**Figure 3** shows a circuit diagram of a microelectrode pixel 1 of the microelectrode arrangement according to the invention. Each microelectrode pixel 1 integrates a contacting electrode 2 and a simple amplifier circuit 3.

As shown in the schematic cross-section on the left-hand part of Figure 3, the contacting electrode 2 with its active surface 21 is preferably a noble-metal, e.g., gold, deposition on a CMOS-circuit Aluminum-based metal layer 22. It is surrounded by an insulator 23, e.g., Si(x)Ni(y) deposited by Plasma-Enhanced Chemical Vapor Deposition (PECVD).

The amplifier 31 is preferably a programmable differential amplifier with a pair of inputs +, - and an output 32. A non-inverting input + of the amplifier 31 is directly connected to the contacting electrode 2. An inverting input - of the amplifier 31 is connected to the reference electrode 5 via a reference input line 33.

Transistors MN6 and MP3 work as voltage-controlled resistors. By applying a voltage in the range between a ground voltage V_{SS} and a supply voltage V_{DD} on a switching input line 34, it is possible to change the resistance ratio of the transistors MN6 and MP3, and thus to choose between open-loop amplification and closed-loop amplification. Transistor MN5 can be used as a voltage-controlled resistor for amperometric measurements, or as a resetting switch for the integration mode, to discharge a total effective input capacitance C. Transistor MN7 is a MOS output capacitor serving to decrease the signal bandwidth. The low local capacitance in the amplification circuit and the MOS output capacitor (transistor MN7) reduce the signal noise, apart from the short distance between the contacting electrode 2 and the amplifier 31.

The output 32 of the amplifier 31 is connected to an output node 35. Upon addressing of the pixel 1, an output line 36 outputs the in-pixel-amplified signal from the output node 35, e.g., on a column bus and finally on a chip output (not shown).

The amplifier circuits 3 are preferably manufactured in standard CMOS technology. The contacting electrodes 2 are then deposited in a non-standard post-processing step.

The integrated amplifier circuit 3 allows measurements with a programmable low-pass filtering and programmable gain allowing noise/speed trade-off. The gain as well as the DC bandwidth can be externally programmed by varying the DC polarization on the amplifier input pair +, - by means of the DC polarization electrode 6.

According to the invention, the following three different working modes of the amplifier circuit 3 can be selected:

### A. Potentiometric measurements:

The voltage difference between the contacting electrode 2 and the reference electrode 5 connected to a reference input line 33 is amplified by the differential amplifier 31 and outputted on the output node 35. Gate 37 and source 38 of transistor MN5 are connected to the ground voltage V_{SS}. The amplifier circuit 3 can be used in open loop by setting the switching input line 34 to the supply voltage V_{DD}, or as a unity-gain-feedback amplifier by setting the switching input line 34 to the ground voltage V_{SS}. Moreover, by applying a voltage to the switching input line 34 in the range between the ground voltage V_{SS} and the supply voltage V_{DD} it is possible to vary the resistances given by transistors MN6 and MP3, resulting in a feedback-amplified configuration.

### B. Amperometric measurements:

The local current at the contacting electrode 2 can be directly measured using transistor MN5 as a resistor. Its resistance can be controlled by applying a voltage between gate 37 and source 38 of transistor MN5. The resulting voltage drop on the amplifier input pair +, - is amplified as described for potentiometric measurements and outputted on the output node 35.

### C. Charge integration measurements for ultra-low-noise current detection (Integration Mode):

This working mode is explained with reference to **Figures 3 and 4**. In the upper half of Figure 4, a voltage applied to the gate 37 of transistor MN5 is plotted versus time t; in the lower half, the voltage at the output node 35 is plotted versus time t.
A small capacitance C at the non-inverting input + of the amplifier 31 is charged by the electrode current, generating a voltage difference at the amplifier input pair +, -. For very small capacitance values C the stray capacitance of the circuit 3 can be used without the explicit integration of an additional capacitor device. After a pre-defined or known short time interval Δt (Figure 3), the capacitor C is discharged by transistor MN5 and the amplified voltage Vₚ = C/Q is read out at the output node 35, where Q = I·Δt. The capacitor C is reset for the next charge integration period by making transistor MN5 conductive. This is achieved by applying the supply voltage V_{DD} to the gate 37 and the ground voltage V_{SS} to the source 38 of transistor MN5. Since the amplifier 31 is very sensitive to DC voltage signals on its input pair +, -, the integration mode makes possible the measurements of very low currents at the contacting electrode 2 with ultra-low noise.

Pixels with other functionality, e.g., temperature sensors, pH sensors, integrated references and/or electrically stimulating pixels, can be added to the active microelectrode array according to the invention.

### List of reference signs

- 1: Microelectrode pixel

- 2: Contacting electrode
- 21: Active surface
- 22: CMOS-circuit metal layer
- 23: Insulator

- 3: Amplifier circuit
- 31: Amplifier
- 32: Amplifier output
- 33: Reference input line
- 34: Switching input line
- 35: Output node
- 36: Output line
- 37: Gate of transistor MN5
- 38: Source of transistor MN5

- 41, 42: Means for individually addressing and reading out the microelectrode pixels

- 5: Reference electrode

- 6: DC polarization elctrode

- 7: Culture medium

- 8: Chamber

- C: Capacitor
- MP3, MN5, MN6, MN7: Transistors
- t: Time
- V_{DD}: Supply voltage
- V_{SS}: Ground voltage

## Claims

1. A microelectrode arrangement comprising
a plurality of microelectrode pixels (1; 1.11, 1.12, ..., 1.21, 1.22, ...), each microelectrode pixel (1; 1.11, 1.12, ... , 1.21, 1.22, ...) being a basic element of the microelectrode arrangement, different from an optical picture element, and each microelectrode pixel comprising
a contacting electrode (2) being adapted for electrically contacting an external surface (7) and an amplifier circuit (3) having an amplifier (31) for amplifying an electric input signal applied to said contacting electrode (2) from the external surface (7) and means (MN5, 37, 38) configured such that the amplifier circuit is adapted to work selectively in an amperometric measurement mode and in a potentiometric measurement mode in order to measure selectively a current and a voltage corresponding to said electric input signal applied to said contacting electrode (2) from the external surface (7),
**characterized in that**
said means (MN5, C, 37, 38) are also configured such that the amplifier circuit is further adapted to work selectively in a charge integration measurement mode in order to measure charge corresponding to said electric input signal applied to said contacting electrode (2) from the external surface (7) for an ultra-low-noise current detection.

2. The microelectrode arrangement according to claim 1, further comprising means (41, 42) for individually addressing and reading out each microelectrode pixel (1; 1.11, 1.12, ..., 1.21, 1.22, ...) .

3. The microelectrode arrangement according to any of the preceding claims, wherein said amplifier (31) is a differential amplifier and preferably a programmable differential amplifier.

4. The microelectrode arrangement according to any of the preceding claims, wherein said amplifier (31) has at least one amplifier input (+, -) and an amplifier output (32), an amplifier input (+) being connected to said contacting electrode (2) and said amplifier output (32) being connected to an output node (35) of the microelectrode pixel (1; 1.11, 1.12, ..., 1.21, 1.22, ...).

5. The microelectrode arrangement according to claim 4, wherein each microelectrode pixel (1; 1.11, 1.12, ..., 1.21, 1.22, ...) further comprises means (MP3, MN6) for feeding back an electric signal from said amplifier output (32) to an amplifier input (-), and preferably means (34) for varying the resistance of said feedback means (MP3, MN6).

6. The microelectrode arrangement according to any of the claims 4 or 5, wherein each microelectrode pixel (1; 1.11, 1.12, ...., 1.21, 1.22, ...) further comprises means (MN5) for leading away at least part of a current that flows between said contacting electrode (2) and said amplifier input (+), and means (37, 38) for varying the resistance of said current-leading means (MN5).

7. The microelectrode arrangement according to claim 6, wherein each microelectrode pixel (1; 1.11, 1.12, ... , 1.21, 1..22, ...) further comprises a capacitor (C) connected to said amplifier input (+).

8. The microelectrode arrangement according to any of the claims 4-7, wherein said output node (35) is connected to a capacitor (MN7) for decreasing the output-signal bandwidth.

9. The microelectrode arrangement according to any of the claims 4-8, further comprising a reference electrode (5) that is preferably connected to a further amplifier input (-) in each microelectrode pixel (1; 1.11, 1.12, ..., 1.21, 1.22, ...).

10. The microelectrode arrangement according to any of the preceding claims, further comprising a DC polarization electrode (6) for controlling the DC polarization of said amplifier (31).

11. The microelectrode arrangement according to any of the preceding claims, wherein said amplifier (31) is manufactured in complementary metal oxide semiconductor technology.

12. The microelectrode arrangement according to any of the preceding claims, wherein said microelectrode pixels (1; 1.11, 1.12,..., 1.21, 1.22, ...) are arranged in rows and columns of a two-dimensional array.

13. The microelectrode arrangement according to any of the preceding claims, further comprising pixels with other functionality than said microelectrode pixels, such as temperature-sensor pixels, pH-sensor pixels, integrated-reference pixels and/or electrically stimulating pixels.

## Patentansprüche

1. Mikroelektrodenanordnung, umfassend:
mehrere Mikroelektrodenpixel (1; 1.11, 1.12, ..., 1.21, 1.22, ...), wobei jedes Mikroelektrodenpixel (1; 1.11, 1.12, ..., 1.21, 1.22, ...) ein Basiselement der Mikroelektrodenanordnung ist, unterschieden von einem optischen Bildelement, wobei jedes Mikroelektrodenpixel umfasst:
eine Kontaktelektrode (2), welche angepasst ist, zum elektrischen Kontaktieren einer äußeren Fläche (7), und eine Verstärkerschaltung (3), welche einen Verstärker (31) aufweist, zum Verstärken eines elektrischen Eingangssignals, welches auf die Kontaktelektrode (2) angelegt wird, von der äußerem Fläche (7), und Mittel (MN5, 37, 38), welche derart konfiguriert sind, dass die Verstärkerschaltung angepasst ist, wahlweise in einem amperometrischen Messmodus und einem potentiometrischen Messmodus zu arbeiten, um wahlweise einen Strom und eine Spannung zu messen, entsprechend dem elektrischen Eingangssignal, welches auf die Kontaktelektrode (2), von der äußeren Fläche (7), angelegt wird,
**dadurch gekennzeichnet, dass**
die Mittel (MN5, C, 37, 38) ebenfalls derart konfiguriert sind, dass die Verstärkerschaltung ferner angepasst ist, wahlweise in einem ladungsintegrierenden Messmodus zu arbeiten, um Ladung, entsprechend dem elektrischen Eingangssignal, zu messen, welches auf die Kontaktelektrode (2), von der äußeren Fläche (7) angelegt wird, für eine extrem rauscharme Stromdetektion.

2. Mikroelektrodenanordnung nach Anspruch 1, ferner umfassend Mittel (41, 42), zum einzelnen Adressieren und Auslesen jedes Mikroelektrodenpixels (1; 1.11, 1.12, ..., 1.21, 1.22, ...).

3. Mikroelektrodenanordnung nach einem der vorhergehenden Ansprüche, wobei der Verstärker (31) ein Differenzverstärker, und vorzugsweise ein (programmierbarer) Differenzverstärker, ist.

4. Mikroelektrodenanordnung nach einem der vorhergehenden Ansprüche, wobei der Verstärker (31) wenigstens einen Verstärkereingang (+, -) und einen Verstärkerausgang (32) aufweist, wobei ein Verstärkereingang (+) mit der Kontaktelektrode (2) verbunden ist, und der Verstärkerausgang (32) mit einem Ausgangsknoten (35) des Mikroelektrodenpixels (1; 1.11, 1.12, ..., 1.21, 1.22, ...) verbunden ist.

5. Mikroelektrodenanordnung nach Anspruch 4, wobei jedes Mikroelektrodenpixel (1; 1.11, 1.12, ..., 1.21, 1.22, ...) ferner Mittel (MP3, MN6) umfasst, zum Rückkoppeln eines elektrischen Signals, aus dem Verstärkerausgang (32) zu einem Verstärkereingang (-), und vorzugsweise Mittel (34), zum Variieren des Widerstands der Rückkopplungsmittel (MP3, MN6).

6. Mikroelektrodenanordnung nach einem der Ansprüche 4 oder 5, wobei jedes Mikroelektrodenpixel (1; 1.11, 1.12, ..., 1.21, 1.22, ...) ferner Mittel (MN5) umfasst, zum Abführen wenigstens eines Teils eines Stroms, welcher zwischen der Kontaktelektrode (2) und dem Verstärkereingang (+) fließt, und Mittel (37, 38), zum Variieren des Widerstands der Stromführungsmittel (MN5).

7. Mikroelektrodenanordnung nach Anspruch 6, wobei jedes Mikroelektrodenpixel (1; 1.11, 1.12, ..., 1.21, 1.22, ...) ferner einen Kondensator (C) umfasst, verbunden mit dem Verstärkereingang (+).

8. Mikroelektrodenanordnung nach einem der Ansprüche 4 bis 7, wobei der Ausgangsknoten (35) mit einem Kondensator (MN7) verbunden ist, zum Verringern der Ausgangssignalbandbreite.

9. Mikroelektrodenanordnung nach einem der Ansprüche 4 bis 8, ferner umfassend eine Referenzelektrode (5), welche vorzugsweise mit einem weiteren Verstärkereingang (-) verbunden ist, in jedem Mikroelektrodenpixel (1; 1.11, 1.12, ..., 1.21, 1.22, ...).

10. Mikroelektrodenanordnung nach einem der vorhergehenden Ansprüche, ferner umfassend eine Gleichstrompolarisierungselektrode (6), zum Steuern der Gleichstrompolarisierung des Verstärkers (31).

11. Mikroelektrodenanordnung nach einem der vorhergehenden Ansprüche, wobei der Verstärker (31) in CMOS-(Complementary-Metal-Oxide-Semiconductor-)Halbleitertechnologie hergestellt ist.

12. Mikroelektrodenanordnung nach einem der vorhergehenden Ansprüche, wobei die Mikroelektrodenpixel (1; 1.11, 1.12, ..., 1.21, 1.22, ...) in Reihen und Spalten eines zweidimensionalen Arrays angeordnet sind.

13. Mikroelektrodenanordnung nach einem der vorhergehenden Ansprüche, ferner umfassend Pixel mit anderer Funktionalität als die Mikroelektrodenpixel, wie etwa Temperatursensorpixel, pH-Sensorpixel, integrierte Referenzpixel, elektrisch stimulierende Pixel.

## Revendications

1. Agencement de microélectrode comprenant :
une pluralité de pixels de microélectrode (1; 1.11, 1.12, ..., 1.21, 1.22, ...), chaque pixel de microélectrode (1; 1.11, 1.12, ..., 1.21, 1.22, ...) étant un élément de base de l'agencement de microélectrode, différent d'un élément d'image optionnel, et chaque pixel de microélectrode comprenant
une électrode de contact (2) étant adaptée pour contacter électriquement une surface externe (7) et un circuit d'amplification (3) comportant un amplificateur (31) pour amplifier un signal d'entrée électrique appliqué à ladite électrode de contact (2) à partir de la surface externe (7) et des moyens (MN5, 37, 38) configurés de telle sorte que le circuit d'amplification soit adapté afin de fonctionner sélectivement en un mode de mesure ampérométrique et en un mode de mesure potentiométrique de manière à mesurer sélectivement un courant et une tension correspondant audit signal d'entrée électrique appliqué à ladite électrode de contact (2) à partir de la surface externe (7),
**caractérisé en ce que**
lesdits moyens (MN5, C, 37, 38) sont aussi configurés de telle sorte que le circuit d'amplification soit en outre adapté afin de fonctionner sélectivement en un mode de mesure d'intégration de charge de manière à mesurer une charge correspondant audit signal d'entrée électrique appliqué à ladite électrode de contact (2) à partir de la surface externe (7) en vue d'une détection de courant à bruit ultra-bas.

2. Agencement de microélectrode selon la revendication 1, comprenant en outre des moyens (41, 42) pour adresser et lire individuellement chaque pixel de microélectrode (1; 1.11, 1.12, ..., 1.21, 1.22, ...) .

3. Agencement de microélectrode selon une quelconque des revendications précédentes, dans lequel ledit amplificateur (31) est un amplificateur différentiel et de préférence un amplificateur différentiel programmable.

4. Agencement de microélectrode selon une quelconque des revendications précédentes, dans lequel ledit amplificateur (31) possède au moins une entrée d'amplificateur (+,-) et une sortie d'amplificateur (32), une entrée d'amplificateur (+) étant connectée à ladite électrode de contact (2) et ladite sortie d'amplificateur (32) étant connectée à un noeud de sortie (35) du pixel de microélectrode (1; 1.11, 1.12, ..., 1.21, 1.22, ...) .

5. Agencement de microélectrode selon la revendication 4, dans lequel chaque pixel de microélectrode (1; 1.11, 1.12, ..., 1.21, 1.22, ...) comprend en outre des moyens (MP3,MN6) pour réinjecter un signal électrique depuis ladite sortie d'amplificateur (32) vers une entrée d'amplificateur (-) et de préférence, des moyens (34) pour varier la résistance dudit moyen de réinjection (MP3,MN6).

6. Agencement de microélectrode selon une quelconque des revendications 4 ou 5, dans lequel chaque pixel de microélectrode (1; 1.11, 1.12, ..., 1.21, 1.22, ...) comprend en outre des moyens (MN5) pour dévier au moins une partie d'un courant qui circule entre ladite électrode de contact (2) et ladite entrée d'amplificateur (+) et des moyens (37, 38) pour varier la résistance dudit moyen de déviation de courant.

7. Agencement de microélectrode selon la revendication 6, dans lequel chaque pixel de microélectrode (1; 1.11, 1.12, ..., 1.21, 1.22, ...) comprend en outre un condensateur (C) connecté à ladite entrée d'amplificateur (+).

8. Agencement de microélectrode selon une quelconque des revendications 4 à 7, dans lequel ledit noeud de sortie (35) est connecté à un condensateur (MN7) pour diminuer la largeur de bande du signal de sortie.

9. Agencement de microélectrode selon une quelconque des revendications 4 à 8, comprenant en outre une électrode de référence (5) qui est de préférence connectée à une autre entrée d'amplificateur (-) dans chaque pixel de microélectrode (1; 1.11, 1.12, ..., 1.21, 1.22, ...) .

10. Agencement de microélectrode selon une quelconque des revendications précédentes, comprenant en outre une électrode de polarisation à courant direct (6) pour commander la polarisation à courant direct dudit amplificateur (31).

11. Agencement de microélectrode selon une quelconque des revendications précédentes, dans lequel ledit amplificateur (31) est fabriqué selon une technologie de semi-conducteur à oxyde métallique complémentaire.

12. Agencement de microélectrode selon une quelconque des revendications précédentes, dans lequel lesdits pixels de microélectrode (1; 1.11, 1.12, ..., 1.21, 1.22, ...) sont agencés en lignes et en colonnes dans une matrice en deux dimensions.

13. Agencement de microélectrode selon une quelconque des revendications précédentes, comprenant en outre des pixels ayant d'autres fonctionnalités que lesdits pixels de microélectrode, comme des pixels de capteur de température, des pixels de capteur de pH, des pixels de référence intégrés et/ou des pixels de stimulation électrique.
